# EUROPÄISCHE PATENTSCHRIFT

(11) **EP 3 386 501 B1**
(45) Veröffentlichungstag und Bekanntmachung des Hinweises auf die Patenterteilung: **28.06.2023**
(21) Anmeldenummer: 16819796.0
(22) Anmeldetag: 08.12.2016
(51) Int. Cl.: A61K 31/40, A61K 31/44, A61K 31/135, A61K 31/485, A61K 9/51, A61P 25/04

(54) **ANALGETISCHE ZUSAMMENSETZUNGEN MIT NANOCARRIERN UND IHRE VERWENDUNG**
ANALGESIC PREPARATION WITH NANOCARRIERS AND USE THEREOF
COMPOSITIONS ANALGÉSIQUES CONTENANT DES NANOVECTEURS ET UTILISATION DESDITES COMPOSTIONS

(30) Priorität: 08.12.2015 DE 102015121366
(43) Veröffentlichungstag der Anmeldung: 17.10.2018
(73) Patentinhaber: DendroPharm GmbH, 12107 Berlin (DE)
(72) Erfinder: FLEIGE, Emanuel, 12163 Berlin (DE); MORÉ, Sam, 01109 Dresden (DE); UNBEHAUEN, Michael, 12053 Berlin (DE); HAAG, Rainer, 12209 Berlin (DE); STEIN, Christoph, 12203 Berlin (DE); MACHELSKA, Halina, 12203 Berlin (DE); PAULUS, Florian, 14195 Berlin (DE)
(74) Vertreter: Moré, Solveig Helga
(86) Internationale Anmeldenummer: PCT/DE2016/100569
(87) Internationale Veröffentlichungsnummer: WO 2017/097291

(56) Entgegenhaltungen:
- WO-A2-2006/018295
- WO-A2-2009/123934
- WO-A2-2011/011384
- DE-A1-102014 008 575
- BRENT B WARD ET AL: "Sustained Analgesia Achieved Through Esterase-Activated Morphine Prodrugs Complexed with PAMAM Dendrimer", PHARMACEUTICAL RESEARCH, KLUWER ACADEMIC PUBLISHERS-PLENUM PUBLISHERS, NL, Bd. 30, Nr. 1, 28. September 2012 (2012-09-28), Seiten 247-256, XP035152040, ISSN: 1573-904X, DOI: 10.1007/S11095-012-0869-3
- CARRASCO-SÁNCHEZ VERÓNICA ET AL: "In situandin silicoevaluation of amine- and folate-terminated dendrimers as nanocarriers of anesthetics", EUROPEAN JOURNAL OF MEDICINAL CHEMISTRY, Bd. 73, 12. Dezember 2013 (2013-12-12), Seiten 250-257, XP028608884, ISSN: 0223-5234, DOI: 10.1016/J.EJMECH.2013.11.040
- MARCELO CALDERÓN ET AL: "Dendritic Polyglycerols for Biomedical Applications", ADVANCED MATERIALS, Bd. 22, Nr. 2, 7. Oktober 2009 (2009-10-07), Seiten 190-218, XP055015111, ISSN: 0935-9648, DOI: 10.1002/adma.200902144

## Beschreibung

Die Erfindung betrifft das Gebiet der Analgetika. Es wird ein Kit oder eine pharmazeutische Zusammensetzung zur Verwendung bei der Behandlung von akuten und chronischen Schmerzen und/oder Entzündungen durch periphere Wirkung eines Analgetikums zur Verfügung gestellt, wobei die Wirkung des Analgetikums im Zentralnervensystem im Vergleich zu einer entsprechenden Zusammensetzung ohne Nanocarrier reduziert ist. Die Zusammensetzung oder das Kit umfassen einen Nanocarrier, welcher ein hochverzweigtes Polymer ausgewählt aus der Gruppe umfassend dendritische Polymere und Dendrimere umfasst, wobei der dendritische Kern des Nanocarriers aus Polyglycerol ist und ein Molekulargewicht von 3-10 kDa hat, und ein definiertes Opioid-Analgetikum, welches bei Verabreichung ohne Nanocarrier sowohl periphere als auch analgetische Wirkung im zentralen Nervensystem (ZNS) aufweist. Erfindungsgemäß weist das Analgetikum in dieser Zusammensetzung hauptsächlich oder ausschließlich periphere Wirkung auf und reduziert damit Nebenwirkungen, welche durch die Aktivierung zentraler oder intestinaler Opioidrezeptoren ausgelöst werden.

Die Aktivierung von Opioidrezeptoren in verletztem oder entzündetem peripheren Gewebe kann Schmerzen reduzieren. Durch Aktivierung von Opioidrezeptoren im intestinalen oder zentralen Nervensystem (zentrale Wirkung) werden jedoch Nebenwirkungen wie Konstipation, Sedierung, Apnoe oder Suchterzeugung ausgelöst. Dadurch ist die Verwendbarkeit von konventionellen Opioiden zur Schmerzlinderung eingeschränkt. Formulierungen von Opioid-Agonisten für die systemische Verabreichung, die im Wesentlichen peripher wirksam sind, sind bislang nicht für Patientenbehandlung verfügbar.

Konventionelle Opioidrezeptor-Agonisten wie z.B. Morphin sind der Goldstandard für die Behandlung starker Schmerzen. Jedoch ist deren Einsatz mit Nebenwirkungen verbunden, welche im Wesentlichen durch Permeation der Blut-Hirn-Schranke (BHS) und schließlich durch Aktivierung von Opioidrezeptoren im ZNS führt. Die Folgen sind u.a. Sedierung, Apnoe, Toleranz, Abhängigkeit, Missbrauch oder Überdosierung mit Todesfolge [Schumacher, M.A. et al., Basic and clinical pharmacology (eds. Katzung, B.G., Masters, S.B. & Trevor, A.J.) 531-552 (McGraw-Hill Medical, New York, 2009), Paulozzi, L.J. et al., J Safety Res 43, 283-289 (2012)]. Durch Aktivierung von intestinalen Opioidrezeptoren im nicht-entzündeten (unverletzten) Magen-Darm Trakt kann ausserdem Konstipation ausgelöst werden. Nichtsteroidale entzündungshemmende Medikamente sind Alternativen, aber auch sie verursachen schädliche Nebenwirkungen wie Magen-Darm-Blutungen, Geschwüre oder kardiovaskuläre Komplikationen (Lancet 382:769-79, 2013). Neue Schmerzmittel sind demnach dringend erforderlich.

Konventionelle stärkere mu-Opioidrezeptor-Agonisten wie z.B. Morphin oder Oxycodon besitzen durch ihre euphorisierende Wirkung ein signifikantes Missbrauchs- und Suchtpotential. Aktueller Stand der Technik ist daher eine Kombination mit einem partiellen kappa-Opioidrezeptor-Agonisten oder einem Antagonisten wie Naloxon. Beispiele dafür sind Oxycodon/Naloxon Kombinationspräparate. Oxycodon (ATC N02AA55) ist ein halbsynthetisches Opioid mit schmerzlindernden Eigenschaften. Naloxon ist ein kompetitiver Antagonist an allen Opioidrezeptoren, der in der hier verwendeten Dosierung die Wirkung von Oxycodon lokal im Darm aufhebt und damit die Entstehung einer Verstopfung verhindern soll. Trotzdem benötigen noch ca. 50% aller Patienten, die mit Oxycodon/Naloxon Kombinationen behandelt werden eine unterstützende Therapie mit Abführmitteln zur Verhinderung einer schweren Konstipation. In höherer Dosierung können auch mit dieser Kombination über zentrale mu-Opioidrezeptoren vermittelte euphorisierende (suchterzeugende) Effekte nicht vollständig verhindert werden.

Eine Alternative zu mu-Opioidrezeptor-Agonisten könnten kappa- Opioidrezeptor-Agonisten darstellen. Nachdem in den 80er Jahren des vergangenen Jahrhundert erste Ergebnisse mit sogenannten kappa-Agonisten dieses Ziel zunächst in greifbare Nähe rücken ließen, stellten sich bald jedoch heraus, dass diese Stoffe beim Überwinden der BHS nicht nur eine Aversion gegen das Medikament auslösten, sondern auch Halluzinationen, Sedierung und Depressions-ähnliche Symptome. Aufgrund dieser ausgeprägten Nebenwirkungen gibt es nur wenige Schmerzmittel wie z.B. Butorphanol sowie Buprenorphin, die auf einer zumindest partiellen kappa-Opioidrezeptor-agonistischen Wirkung beruhen. Die ausgeprägte dysphorische und halluzinogene Wirkung insbesondere von kappa-Agonisten verhindert zwar eine Suchtausbildung und Medikamentenmissbrauch, stellt jedoch gleichzeitig eines der Haupthindernisse für eine analgetische Anwendung dar.

Es wurden bereits mizellare Nanocarrier-Opioid-Formulierungen entwickelt, wie zum Beispiel liposomales Morphin. Die Haupteigenschaft dieser Formulierungen ist eine Retardierung der Wirkdauer sowie die Erhöhung der Löslichkeit des Wirkstoffes. Liposomale Träger und Nanocarrier aus amphiphilen Mizellen sind allgemein dafür bekannt, dass sie den Wirkstoff-Übertritt über die BHS erhöhen.

WO 2009/1239234 A2 offenbart hochverzweigte Polyglycerin-Polymere, die mit Wirkstoffen beladen werden können. Die Liste möglicher Wirkstoffe ist äußerst vielseitig und umfasst unter anderem Opiate wie die mu-Opioidrezeptor-Agonisten Codein, Morphin und Analoga davon. Für eine zielgerichtete Wirkstofffreigabe sollen die Nanocarrier mit diversen Targeting-Reagenzien modifiziert werden.

Opioidrezeptoren sind nicht nur im Gehirn lokalisiert, sondern werden auch in peripheren sensorischen Neuronen gebildet und können deren Erregung durch schmerzhafte Reize blockieren [Stein, C. et al., Proc Natl Acad Sci U S A 87, 5935-5939 (1990); Moshourab, R. et al., J Neurophysiol 108, 2827-2836 (2012); Nockemann, D. et al., EMBO Mol Med 5, 1263-1277 (2013); Stein, C. et al., Pharmacol Rev 63, 860-881 (2011)]. Diese Rezeptoren werden in peripherem, verletztem Gewebe hochreguliert und können selektiv und zielgerichtet mit Opioid-Agonisten angesteuert werden, um zentrale oder intestinale Nebenwirkungen zu vermeiden [Stein, C. et al., Pharmacol Rev 63, 860-881 (2011); Kalso , E. et al., Pain 98, 269-275 (2002); Vadivelu, N. et al., J Opioid Manag 7, 55-68 (2011); Stein, C. et al., N Engl J Med 325, 1123-1126 (1991); Stein, C. et al., Trends Pharmacol Sci 34, 303-312 (2013); Stein, C. et al., Nat Med 9, 1003-1008 (2003); Stein C. N Engl J Med., 332, 1685-90 (1995)]. Tatsächlich konnte bereits gezeigt werden, dass Schmerzen und Entzündungen im Tiermodell sowie postoperative und arthritische Schmerzen beim Menschen durch lokale Applikation von geringen, systemisch inaktiven Dosen von Opioid-Agonisten effizient verringert werden können [Kalso , E. et al., Pain 98, 269-275 (2002); Stein, C. et al., Pharmacol Rev 63, 860-881 (2011)]. Allerdings sind Formulierungen von selektiv peripher wirksamen Opioid-Agonisten für die systemische (z. B. intravenöse) Verabreichung bislang nicht allgemein verfügbar. Bisherige Strategien verwendeten polare Opioid-Liganden, um die BHS-Permeabilität zu verringern oder z.B. Antikörper-Konjugate für die gezielte gewebespezifische Freisetzung von Wirkstoffen [Stein, C. Anesth Analg 76, 182-191 (1993); Hua, S. et al., Pain Physician 16, E199-216 (2013); Cheng, Z. et al., Science 338, 903-910 (2012)]. Die Verwendung von polaren Opioid-Liganden war jedoch weitgehend erfolglos, weil polare Reste die Affinität der Liganden zu Opioidrezeptoren verringern und die vollständige Undurchlässigkeit der BHS bei höheren Dosen von Liganden nicht gegeben war [Stein, C. et al., Pharmacol Rev 63, 860-881 (2011); Brown, D.R. et al., Neuropharmacology 24, 181-191 (1985)]. Antikörper-Konjugate haben Probleme wie allergische Reaktionen, eine niedrige Wirkstoff-Beladung, Kreuzreaktivität mit gesundem oder off-Target-Gewebe, Beeinträchtigung der Freisetzung von aktiven Wirkstoffen, rasche Ausscheidung oder hohe molekulare Komplexität [Baker, M. et al., Curr Drug Saf 5, 308-313 (2010); Ovadia, O. et al., Expert Opin Drug Discov 5, 655-671 (2010); Koshkaryev, A. et al., Adv Drug Deliv Rev 65, 24-35 (2013); Cheng, Z. et al., Science 338, 903-910 (2012)].

Dem gegenüber stellten sich die Erfinder die Aufgabe, eine verbesserte Zusammensetzung zur Verfügung zu stellen, welche bei geringen oder fehlenden zentralen Nebenwirkungen schmerzlindernde Wirkung aufweist, wobei die genannten Probleme vermindert oder vermieden werden. Dieses Problem wird durch die Erfindung, insbesondere durch den Gegenstand der Ansprüche, gelöst.

Die Erfindung stellt eine Zusammensetzung zur Verfügung, umfassend
- einen hydrophilen Nanocarrier, welcher ein hochverzweigtes Polymer ausgewählt aus der Gruppe umfassend dendritische Polymere und Dendrimere umfasst, wobei der dendritische Kern des Nanocarriers aus Polyglycerol ist und ein Molekulargewicht von 3-10 kDa hat, und
- ein Analgetikum, welches bei Verabreichung ohne Nanocarrier sowohl periphere als auch zentrale analgetische Wirkung aufweist, welches ausgewählt ist aus der Gruppe bestehend aus Nalbuphin, Tramadol, Tilidin, Pethidin, Codein, Piritramid, Levomethadon, Fentanyl, Alfentanil, Remifentanil, Sufentanil, Pentazocin, Butorphanol, Buprenorphin, Methadon, 2-(3,4-dichlorophenyl)-N-methyl-N-[(1R,2R)-2-pyrrolidin-1-ylcyclohexyl]acetamid, (+)-(5α,7α,8β)-N-Methyl-N-[7-(1-pyrrolidinyl)-1-oxaspiro[4.5]dec-8-yl]-2-Phenylacetamid, Dimethyl 3,7-dimethyl-9-oxo-2,4-dipyridin-2-yl-3,7-diazabicyclo[3.3.1]nonane-1,5-dicarboxylat, 2-(3,4-dichlorophenyl)-1-[(2S)-2-(pyrrolidin-1-ylmethyl)piperidin-1-yl]ethanon, Bremazocine und Moxazozine,
zur Verwendung bei der Behandlung von akuten und chronischen Schmerzen und/oder Entzündungen durch periphere Wirkung des Analgetikums, wobei die Wirkung des Analgetikums im Zentralnervensystem im Vergleich zu einer entsprechenden Zusammensetzung ohne Nanocarrier reduziert ist.

In einer Ausführungsform der Erfindung sind Analgetikum und Nanocarrier nicht kovalent miteinander assoziiert. Dabei ist es zum einen möglich, dass Analgetikum und Nanocarrier über eine nicht-kovalente Verbindung miteinander assoziiert sind, wobei die nicht-kovalente Verbindung ionische Wechselwirkungen (z.B. zwischen geladenen Resten wie COO⁻ oder NH₃⁺), Van-der-Waals Wechselwirkungen und/oder Wechselwirkungen aromatischer pi-Elektronen umfassen. Insbesondere Nanocarrier mit Kern-Schale-Struktur (mit einer Schale, einer Doppelschale oder mehreren Schalen) sind für diese Zwecke gut geeignet, da solche Nanocarrier mit hydrophilen oder hydrophoben Gastmolekülen, im Falle der vorliegenden Erfindung, Analgetika, insbesondere Opioiden, beladen werden können.

Zum anderen ist es aber auch möglich, dass Nanocarrier und Analgetikum nur miteinander gemischt werden, ohne dass eine enge Assoziierung besteht. Die Erfindung stellt daher auch ein Kit zur Verfügung, umfassend
- einen hydrophilen Nanocarrier, welcher ein hochverzweigtes Polymer ausgewählt aus der Gruppe umfassend dendritische Polymere und Dendrimere umfasst, wobei der Nanocarrier ein hochverzweigtes dendritisches Polyglycerin-Polymer ausgewählt aus der Gruppe umfassend Kern-Schale-Nanocarrier und Kern-Multischalen-Nanocarrier ist, wobei der dendritische Kern des Nanocarriers aus Polyglycerol ist und ein Molekulargewicht von 3-10 kDa hat, und
- ein Analgetikum, welches bei Verabreichung ohne Nanocarrier sowohl periphere als auch zentrale analgetische Wirkung aufweist und ausgewählt ist aus der Gruppe bestehend aus Nalbuphin, Tramadol, Tilidin, Pethidin, Codein, Piritramid, Levomethadon, Fentanyl, Alfentanil, Remifentanil, Sufentanil, Pentazocin, Butorphanol, Buprenorphin, Methadon, 2-(3,4-dichlorophenyl)-N-methyl-N-[(1R,2R)-2-pyrrolidin-1-ylcyclohexyl]acetamid, (+)-(5α,7α,8β)-N-Methyl-N-[7-(1-pyrrolidinyl)-1-oxaspiro[4.5]dec-8-yl]-2-Phenylacetamid, Dimethyl 3,7-dimethyl-9-oxo-2,4-dipyridin-2-yl-3,7-diazabicyclo[3.3.1]nonane-1,5-dicarboxylat, 2-(3,4-dichlorophenyl)-1-[(2S)-2-(pyrrolidin-1-ylmethyl)piperidin-1-yl]ethanon, Bremazocine und Moxazozine,
zur Verwendung bei der Behandlung von akuten und chronischen Schmerzen und/oder Entzündungen durch periphere Wirkung des Analgetikums, wobei die Wirkung des Analgetikums im Zentralnervensystem im Vergleich zu einer entsprechenden Zusammensetzung ohne Nanocarrier reduziert ist.

Das erfindungsgemäße Kit kann zum einen als Zwischenprodukt für eine erfindungsmäße Zusammensetzung dienen, welche Analgetikum und Nanocarrier kovalent oder, bevorzugt, nicht-kovalent, assoziiert umfasst. Zum anderen können die beiden Bestandteile des Kits auch separat verabreicht werden, wobei in diesem Fall eine im Wesentlichen gleichzeitige Verabreichung erfindungsgemäß die zentralen Nebenwirkungen am meisten reduziert. Dabei kann der Nanocarrier oder das Analgetikum zuerst verabreicht werden, wobei die Verabreichung bevorzugt im Abstand von 0-10 min, 0-5 min oder 1-2 min erfolgt. Bevorzugt werden beide Komponenten auf dem gleichen Weg verabreicht, z.B. beide intravenös, aber auch eine unterschiedliche Applikationsform ist möglich.

In einer anderen Ausführungsform liegen Analgetikum und Nanocarrier in einer Zusammensetzung vor und sind kovalent miteinander assoziiert. Das kann z.B. über eine Veresterung von Carboxylgruppen des Nanocarriers mit Hydroxylgruppen des Analgetikums, bevorzugt Opioids, erfolgen.

Der Durchmesser der Nanocarrier kann im Rahmen der Erfindung bei ca. 3-25 nm liegen, bevorzugt ca. 5-20 nm, am meisten bevorzugt 10-18 nm.

Erfindungsgemäße Nanocarrier sind hydrophil. Bevorzugt tragen sie geladene funktionelle Gruppen, besonders bevorzugt negativ geladene funktionelle Gruppen.

Im Rahmen der Erfindung ist der Nanocarrier ein hochverzweigtes Polymer, bevorzugt ein dendritisches Polymer oder Dendrimer.

Es handelt sich bei den erfindungsgemäßen Nanocarriern also um unimolekulare Nanocarrier. Als Dendrimere bezeichnet man chemische Verbindungen, deren Struktur ausgehend von einem Verzweigungskern gleich einem Baum perfekt verästelt ist. Dabei spricht man von Dendrimeren, wenn diese Verästelungen aus repetitiven Einheiten bestehen, sie somit eine radiale Symmetrie ergeben. Diese müssen also - geht man von einem Kern aus - Verzweigungen enthalten, da man sonst eine Kette erhält. Es kann eine Verzweigung zu zwei oder auch mehr Verknüpfungsstellen geben.

Leichter herzustellen sind bei sehr ähnlichen Eigenschaften dendritische oder hochverzweigte Polymere (hyperbranched polymers), bei denen nicht alle möglichen Verzweigungsstellen verzweigt sind. Bevorzugt handelt es sich bei den Nanocarriern im Rahmen der Erfindung um hochverzweigtes Polyglycerin (hPG) oder einen hochverzweigten Polyester, was Derivate davon einschließt. Diese hochverzweigten Polyglycerine bzw. Polyester besitzen einen Verzweigungsgrad von 25% oder mehr, bevorzugt 25-66% oder 45-66%. Hochverzweigte Polymere unterscheiden sich von Stern-Polymeren mit einem relativ geringen Grad an Verzweigung. Ein Stern-Polymer besitzt ein multifunktionales Zentrum welches mit linearen Polymerketten gleicher oder unterschiedlicher Länge verknüpft ist und einen Verzweigungsgrad von weniger als 25% aufweist. Der Verzweigungsgrad wird nach Hawker et al. (J. Am. Chem. Soc. 1991, 113, 4583-4588) berechnet.

Solche hochverzweigten dendritischen Polyglycerin-Polymere bzw. Polyester-Polymere können z.B. Kern-Schale-Nanocarrier oder Kern-Multischalen-Nanocarrier sein, z.B. mit einer Doppelschale. Dabei werden als Kern bzw. Schale Schichten des Nanocarriers bezeichnet, die sich in ihrer Hydrophilität oder Hydrophobie unterscheiden. Z.B. kann der Kern hydrophob sein und die Schale hydrophil. Ein möglicher Aufbau für einen Kern-Multischalen-Nanocarrier ist z.B. Kern - Esterbindung - Disäure -Esterbindung- mPEG.

Die im Rahmen der Erfindung genutzten Nanocarrier sind hydrophil. Dies kann durch die Anwesenheit von polaren, also hydrophilen und/oder geladenen Gruppen zustande kommen, z.B. Hydroxylgruppen, Carboxylgruppen, Aminogruppen, Sulfatgruppen oder Succinatgruppen. Es kann sich um anionische oder kationische funktionelle Gruppen handeln. Die funktionellen Gruppen können biologisch abbaubar sein. Bei den erfindungsgemäßen Nanocarriern sind bevorzugt 2-50%, mehr bevorzugt 5-15%, der funktionellen endständigen Gruppen mit ionischen Gruppen funktionalisiert, bevorzugt mit negativer Ladung. Dabei können sich diese Gruppen auch im Inneren des Nanocarriers befinden.

In einer Ausführungsform der Erfindung umfasst die erfindungsgemäße Zusammensetzung nicht die in DE 102014008575.9 beschriebene Verbindungen, also nicht ein Konjugat aus einem hydrophilen Polymer wie Polyglycerin mit bioabbaubaren anionischen Endgruppen wie Succinat, die mit einem analgetischen Wirkstoff wie Morphin oder einem anderen Opioidrezeptorliganden z.B. über eine Estergruppe verknüpft sind. In einer anderen Ausführungsform werden entsprechende Konjugate erfindungsgemäß eingesetzt.

In einer bevorzugten Ausführungsform ist der Nanocarrier ein unimolekularer Nanocarrier mit dendritischer Struktur, wobei der Nanocarrier bevorzugt aus einem dendritischen Kern und aus mindestens zwei Schalen besteht, wobei eine innere Schale mit einem ersten Linker mit dem dendritischen Kern verbunden ist und eine äußere Schale mit einem zweiten Linker mit der inneren Schale verbunden ist. In einer Ausführungsform sind die Nanocarrier neutral, kationisch oder anionisch, bevorzugt geladen. Entsprechende Nanocarrier sind z.B. in WO 2006/018295 A2 offenbart. Auch Nanocarrier, die in Radowski et al. (Angew. Chem. Int. Ed. 2007, 46, 1265-1269); Fleige et al. (Macromolecules 2012, 45, 9452-9459), Fleige et al. (Nanocarriers, Vol. 1, 2013, 1-9, WO2011/095311), oder Haag et al., (Macromolecules, 2000, 33, 8158-8166) offenbart sind, können im Rahmen der Erfindung verwendet werden. Ein unimolekularer sulfatierter polyanionischer Nanocarrier, insbesondere ein unimolekulares polyanionische Polyglycerin-Polymer mit einer hydrophilen Schale und einem hydrophoben Kern (EP-Anmeldung Nr. 14 161 579.9) kann ebenfalls eingesetzt werden. Nanocarrier, die sich wie jene Nanocarrier nach systemischer Applikation bevorzugt in entzündeten Arealen anreichern können im Rahmen der vorliegenden Erfindung gut verwendet werden.

Der Nanocarrier ist so ausgestaltet, dass
a) der dendritische Kern des Nanocarriers aus Polyglycerin ist und ein Molekulargewicht von 3-10 kDa, bevorzugt 7-10 oder 8-9 kDa hat.
   Ferner kann
b) die innere Schale des Nanocarriers eine bevorzugt lineare Alkylkette mit einer Kohlenstofflänge von C2 bis C40, bevorzugt C8-C18 oder C12-C15, z.B. C15, sein; und/oder
c) die äußere Schale Polyethylenglykol mit der Strukturformel (-CH₂-CH₂O-)ₙ mit n=3-130, welches endständig eine Methoxygruppe, eine Hydroxylgruppe oder eine Carboxylgruppe, bevorzugt eine Methoxygruppe, trägt, sein; und/oder
d) der erste Linker zwischen Kern und innerer Schale eine Ester oder Amidbindung sein; und/oder
e) der zweite Linker zwischen innerer und äußerer Schale eine Esterbindung sein,
wobei bevorzugt alle Merkmale a-e zutreffen.

Der Nanocarrier kann dendritisches Polyglycerindodecansäurepolyethylenglycolat umfassen. Er kann beispielhaft wie folgt charakterisiert sein:
Nomenklaturformel: hPG₁₀₀₀₀(-NH₂)_{0.7}(C₁₂mPEG₃₅₀)_{1.0 (Mn = 350)}
Alternative Nomenklatur: hPG₁₀₀₀₀(-NH₂)_{0.7}(C₁₂mPEG₆)_{1.0 (6 Wiederholungseinheiten im Mittel)}
hPG10k hat ca. 135 funktionelle Gruppen, davon können ca. 40-80%, z.B. etwa 70% zu Aminen umgesetzt sein (Indexzahl 0.7).

| Molekülbestandteile von innen nach außen: | chemischer Bestandteil Größe | Synthese-Baustein |
|---|---|---|
| 1. Dendritischer Kern | hyperverzweigtes Polyglycerin = hPG | hyperverzweigtes Polyglycerolamin = hPG(-NH₂) |
| | Mₙ = 8000-10000 Da | (Mₙ = 10 kDa, 40-80% Aminierung, insbesondere 65-70 %) |
| | Peptidbindung | |
| 2. Lipophile unpolare Schicht aus gesättigten Fettsäuren | C₁₀ oder C₁₂-Fettsäurekette, insbesondere C₁₂ oder C₁₅ | C₁₀-Disäure: |
| | | 1, 10-decandisäure bzw. |
| | | C₁₂-Disäure: |
| | | 1,12-dodecandisäure |
| | | C 15 Disäure |
| | | 1, 15 pentadecandisäure |
| | Esterbindung | |
| 3. Hydrophile Polyethylenglykolkette | mPEG = Methoxypolyethylenglycol; | mPEG = Methoxypolyethylenglycol; |
| | Mₙ = 350 | Mₙ = 350, 500, 700 oder 1000, insbesondere 350 |

Der Nanocarrier kann aggregieren und bei dieser Aggregation Gastmoleküle, bei der vorliegenden Erfindung das Analgetikum aufnehmen. Unimere und Aggregate stehen im Gleichgewicht, welches sich mit zunehmender Verdünnung Richtung Unimer verschiebt, allerdings mit einer langsamen Kinetik. Zusätzlich kommt es, vor allem abhängig von Polarität und pH-Wert der Umgebung, zu einer Freisetzung der Gastmoleküle aus den Aggregaten. Ein Vorteil der im Patent beschriebenen Verbindungen ist, dass die Viskosität damit hergestellter Zusammensetzungen niedriger als bei Polymeren mit linearen Ketten oder bei geringer verzweigten Sternpolymeren ist.

Im Gegensatz zu mizellar verkapselten Wirkstoffen weisen dendritische Nanocarrier eine erhöhte Scherstabilität und ein signifikant niedrigeren "burst release" auf. Letzterer beschreibt den Mechanismus einer unkontrollierten spontanen Wirkstoffabgabe bei einem Medienwechsel z.B. nach der Injektion. Kovalent an Nanocarrier gebundene Wirkstoffe weisen diesen Nachteil nicht auf, sind jedoch auf Grund der hohen Synthesekosten hauptsächlich bei hochpreisigen Nischenapplikationen wirtschaftlich umsetzbar.

Eine nicht-kovalente Anlagerung des Analgetikums, insbesondere des Opioids ist insbesondere mit hPG-basierten Nanocarriern möglich, welche mit Molekülen funktionalisiert sind, die spezifische Wechselwirkungs-Punkte für Opioide aufweisen, insbesondere von ionischer oder hydrophober Natur, oder Kombinationen davon.

In einer Ausführungsform der Erfindung werden positive geladene Wirkstoffe, z.B. Opioide wie Nalbuphin, nicht-kovalent in den erfindungsgemäßen hochverzweigten Nanocarriern verkapselt. Eine Modulation der Ladung der Nanocarrier kann hierbei eine Modulation der Kinetik der Wirkstofffreigabe erlauben. Bevorzugt wird der Wirkstoff nicht-kovalent verkapselt, während der Nanocarrier, welcher z.B. am Kern oder über einen Linker mit einer eine Aminogruppe umfassenden Verbindung ausgestattet ist und, z.B. bei geeignetem pH-Wert, nicht geladen oder negativ geladen ist, um eine hohe Verkapselungseffizienz zu ermöglichen. Geeignete Nanocarrier sind hierin beschrieben.

Die Ladung des Nanocarriers kann bei einem anderen pH-Werts modifiziert werden. Der Nanocarrier wird dabei so gewählt, dass sich nach Verabreichung der Zusammensetzung bei dem Nanocarrier in entzündetem Gewebe, also bei saurem pH-Wert, eine positive Ladung ergibt. Diese beschleunigt die Freisetzung des ebenfalls positiv geladenen Wirkstoffes.

Alternativ ist es auch möglich, die Freisetzung eines positiv geladenen Wirkstoffs durch Nutzung von im Zielgewebe mit saurem pH negativ geladenen Nanocarriers zu verzögern, was die Herstellung von Sustained release-Zusammensetzungen ermöglicht. Bei einem negativ geladenen Wirkstoff sind entsprechend umgekehrte Ladungen des Nanocarriers auszuwählen.

Auch eine Co-Verkapselung mit hydrophoben ionischen Verbindungen, z.B. langkettigen Aminogruppen tragenden Verbindungen, ist möglich. Dann kann ein Nanocarrier gewählt werden, der auch in entzündetem Gewebe neutral ist. Die verkapselten hydrophoben ionischen Verbindungen werden so gewählt, dass sie in entzündetem Gewebe geladen sind und die Kinetik der Wirkstofffreigabe modulieren, z.B. sind diese Verbindungen dann positiv geladen und beschleunigen die Freigabe eines positiv geladenen Wirkstoffes, etwa eines Opioids wie Nalbuphin.

Die Stärke der Wechselwirkungen zwischen Nanocarrier und Analgetikum kann durch Variation der Anzahl und der Art der mit dem Wirkstoff interagierenden Gruppen genau eingestellt werden.

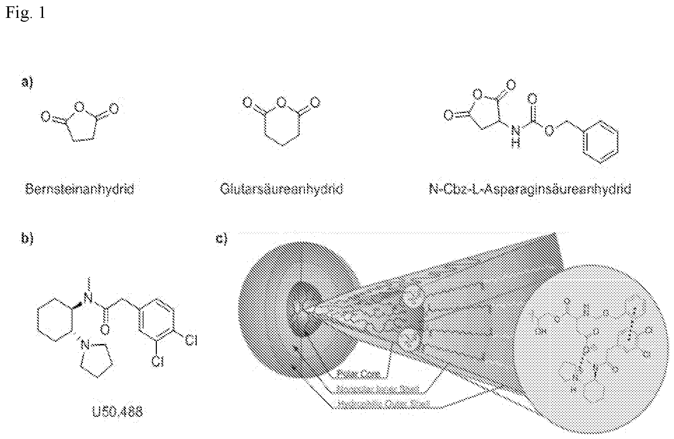

In einer Ausführungsform der Erfindung werden in Verbindung mit dem Analgetikum Nanocarrier zur Verfügung gestellt, welche einen neuartigen ionischen Schlüssel-Schloss Mechanismus aufweisen, der es erlaubt, mit einer Sorte Nanocarrier eine Vielzahl von Wirkstoffen zu verkapseln. Dieses Schlüssel-Schloss Prinzip wird etwa durch die Synthese von CMS (Core-Multishell)-Nanocarriern verwirklicht, die am Kern mit zusätzlichen Ankermolekülen, z.B. freien Säuregruppen ausgestattet sind. Als mögliche Reaktionspartner kommen etwa Bernsteinsäure- und Glutarsäureanhydrid in Frage, da diese eine direkte Reaktion mit dem Kern der Nanocarrier eingehen können (Fig 1a). Ein weiterer interessanter Kandidat sind Derivatisierungen mit Peptiden oder Aminosäuren, z.B. mit N-Cbz-L-Glutaminsäureanhydrid oder N-Cbz-L-Asparaginsäureanhydrid. Diese geschützte, zyklisierte Aminosäure könnte zusätzlich zu der Säuregruppe noch π-π-Wechselwirkungen über den Aromaten der Cbz-Gruppe ermöglichen. Auch Derivatisierungen mit Asparaginsäure, Glutaminsäure, Phenylalanin sind möglich. Als Peptide können z.B. Peptide verwendet werden, welche ***2*-*10*** Aminosäuren enthalten, wobei bevorzugt eine oder mehrere der genannten Aminosäuren umfasst ist.

Der Nanocarrier kann ein Kern-Multischalen-Nanocarrier mit positiver oder negativer Ladung mit einem hochverzweigten dendritischen Polyglycerin-Polymer sein, welcher am Kern oder über einen Linker mit einer eine Aminogruppe umfassenden Verbindung ausgestattet ist.

Solche Verbindung können z.B. sein: Amine, insbesondere primäre, sekundäre, oder tertiäre Amine, und Aminosäuren oder Derivate davon, z.B. Alanin oder Alaninderivate oder Glycin oder Derivate davon wie Dimethylglycin, N-Boc-glycin oder Tritylglycin. Derivate sind bevorzugt mit einem Linker aktivierte Aminosäuren.

Alternativ können im Rahmen der Erfindung neuartige Nanocarrier verwendet werden, welche durch ein Verfahren erhältlich sind, bei dem man Kern-Multischalen-Nanocarrier (Fig. 1c) wie Kern - Esterbindung - Disäure -Esterbindung- m-PEG mit Säuren, Basen oder anderen geeigneten chemischen Reagenzien wie Enzymen, z.B. Esterase unter geeigneten Bedingungen und für eine geeignete Zeit in Kontakt bringt, um die oberste Schale des Nanocarriers partiell zu entfernen. Dies führt zu einem erfindungsgemäßen Nanocarrier mit multiplen negativen Ladungen.

Auch Kern Multischale Nanocarrier des Typs Kern - Amidbindung - Disäure -Esterbindungm-PEG, bei denen die Schale mit dem dendritischen Kern über eine Amidbindung verknüpft sind, können unter geeigneten Bedingungen partiell gespalten werden.
Derart herstellbare Nanocarrier können, wenn man als z.B. Edukt
hPG_{2-10kDa}(-OOC-(CH₂)ₓ-COO-mPEG_{y})ₙ
2-10kDa = hPG-Kerngröße
x = Größe der inneren Schale mit z.B. x = 13 für die C₁₅-Disäure
y = mPEG-Größe z.B. 350 für mPEG350 mit einem Mn von 350 g/mol oder z.B. 6 für die Anzahl der Wiederholungseinheiten
n = Funktionalisierungsgrad des Kerns mit Schale z.B. 70%
verwendet, z.B. folgende Struktur aufweisen:

### Hydrolyse CMS auf reiner Esterbasis:

hPG_{2-10kDa}(-OOC-(CH₂)ₓ-COO-mPEG_{y})ₙ → hPG_{2-10kDa}(-OH)_{b} (-OOC-(CH₂)ₓ-COOH)ₘ ((-OOC-(CH₂)ₓ-COO-mPEG_{y})_{n-b-m}
b = Anzahl freier zusätzlicher OH-Gruppen durch komplette Abspaltung der Schale
m = Anzahl an neuer freier Säuregruppen, durch partielle Hydrolyse der Schale

### Hydrolyse CMS auf Ester- und Amidbasis:

hPG_{2-10kDa}(-NHCO-(CH₂)ₓ-COO-mPEG_{y})ₙ → hPG_{2-10kDa}(-NH₂)ₐ (-NHCO-(CH₂)ₓ-COOH)ₘ ((-NHCO-(CH₂)ₓ-COO-mPEG_{y})ₙ₋ₐ₋ₘ
a = Anzahl freier zusätzlicher Amingruppen durch komplette Abspaltung der Schale
m = Anzahl an neuer freier Säuregruppen, durch partielle Hydrolyse der Schale.

Dabei sind Amine unter physiologischen Bedingungen positiv und Carbonsäuren negativ geladen, was in den Formeln oben zur besseren Übersichtlichkeit nicht dargestellt ist.

Erfindungsgemäß einsetzbare Nanocarrier können mit einem Verfahren zu Herstellung eines Multischalen-Nanocarriers mit positiver oder negativer Ladung hergestellt sein, Schritte umfassend, bei denen man die Ladung durch partielle Spaltung von Esterbindungen oder Amidbindungen erzeugt. Dieses Verfahren kann Teil eines Verfahrens zur Herstellung von Zusammensetzungen oder Kits der Erfindung sein. In einem zweiten Schritt können die hergestellten Nanocarrier dann mit einem Analgetikum nicht-kovalent oder kovalent beladen oder gemischt werden, um eine pharmazeutische Zusammensetzung herzustellen.

Im Rahmen der Erfindung ist das Analgetikum ein Opioid. Vorteilhafterweise weist das Analgetikum eine agonistische oder partiell agonistische Wirkung an einem kappa-Opioid-Rezeptor auf. Das Opioid ist also bevorzugt ein kappa-Rezeptor-Agonist wie Nalbuphin.

In einer Ausführungsform ist das Opioid ein kappa-Rezeptor-Agonist, der an den Nanocarrier kovalent gebunden ist, z.B. an Nanocarrier, wie sie in DE 102014008575.9 beschrieben sind, also ein Konjugat aus einem hydrophilen Polymer wie Polyglycerin mit bioabbaubaren anionischen Endgruppen wie Succinat, die mit einem analgetischen Wirkstoff wie Morphin oder einem anderen Opioidrezeptorliganden z.B. über eine Estergruppe verknüpft sind.

Im Rahmen der Erfindung einsetzbare Opioide sind Tramadol, Tilidin, Pethidin, Codein, Piritramid, Levomethadon, Fentanyl, Alfentanil, Remifentanil, Sufentanil, Pentazocin, Butorphanol, Nalbuphin, Buprenorphin, Methadon, U50488 (2-(3,4-dichlorophenyl)-N-methyl-N-[(1R,2R)-2-pyrrolidin-1-ylcyclohexyl]acetamide), U69593 (+)-(5α,7α,8β)-N-Methyl-N-[7-(1-pyrrolidinyl)-1-oxaspiro[4.5]dec-8-yl]-2-Phenylacetamide), HZ-2 (Dimethyl 3,7-dimethyl-9-oxo-2,4-dipyridin-2-yl-3,7-diazabicyclo[3.3.1]nonane-1,5-dicarboxylate), BRL-52537 (2-(3,4-dichlorophenyl)-1-[(2S)-2-(pyrrolidin-1-ylmethyl)piperidin-1-yl]ethanone) und Bremazocine sowie Moxazozine.. Bevorzugt ist das Opioid U50488, U69593, HZ-2, BRL-52537 oder Bremazocine, am meisten bevorzugt U50488. Bei Verwendung von Nalbuphin im Rahmen der Erfindung ergibt sich ein besonders nützliches Profil von Wirkungen und unerwünschten Wirkungen.

Die erfindungsgemäße Zusammensetzung ist eine pharmazeutische Zusammensetzung. Auch das erfindungsgemäße Kit wird pharmazeutisch eingesetzt und wird dann als pharmazeutische Zusammensetzung bezeichnet.

Es ist auch hierin offenbart, andere Wirkstoffe anstelle analgetischer Wirkstoffe in Kombination mit einem erfindungsgemäßen Nanocarrier zur Verwendung bei der Behandlung einer Krankheit oder eines Krankheitssymptoms durch periphere Wirkung des Wirkstoffs einzusetzen, wobei die Wirkung des Wirkstoffs im Zentralnervensystem im Vergleich zu einer entsprechenden Zusammensetzung ohne Nanocarrier reduziert ist. Somit können durch die Kombination mit dem Nanocarrier sonst durch zentrale Wirkungen des Wirkstoffs vermittelte unerwünschte Wirkungen reduziert oder vermieden werden. Die in solchen Zusammensetzungen enthaltenen Wirkstoffe weisen bei Verabreichung ohne Nanocarrier sowohl periphere Wirkung als auch Wirkung im Zentralnervensystem auf.

Die Krankheit oder das Krankheitssymptom wird im Rahmen der Erfindung durch die periphere Wirkung des Wirkstoffs behandelt. Die Zusammensetzung kann auch in Kit-Form vorliegen.

Der Wirkstoff ist im Rahmen der Erfindung ein Analgetikum, nämlich ein Opioid, und es werden akute und chronische Schmerzen und/oder Entzündungen durch periphere Wirkung des Wirkstoffs behandelt, wobei die Wirkung des Analgetikums im Zentralnervensystem im Vergleich zu einer entsprechenden Zusammensetzung ohne Nanocarrier reduziert ist.

Die pharmazeutische Zusammensetzung oder das pharmazeutische Kit umfasst optional weitere Wirkstoffe und/oder Hilfsstoffe. Z.B. können verschiedene Opioide, z.B. mit einem unterschiedlichen Wirkspektrum an verschiedenen Opioidrezeptoren, oder weitere Analgetika und/oder entzündungshemmende Agentien enthalten sein. Die Zusammensetzung kann in Salzlösung (z.B. 0,9% NaCl) oder Puffer (z.B. Phosphate-buffered saline, pH 7,2-7,4) formuliert sein. Es kann sich um eine Zusammensetzung mit verzögerter Freisetzung handeln, z.B. in Kombination mit Hydrogelen oder Lipogelen, die für eine langfristige Wirkungsabgabe geeignet sind.

Die erfindungsgemäßen Pharmazeutika können zur Verabreichung an einen Menschen formuliert sein. Alternativ kann es sich auch um Veterinärpharmazeutika handeln, die z.B. zur Verabreichung an Haustiere wie Katzen, Hunde, Pferde, Kaninchen, Meerschweinchen oder Hamster, Mäuse oder Ratten formuliert sind.

Die pharmazeutische Zusammensetzung kann zur intravenösen, intramuskulären, intraperitonealen, subkutanen, pulmonalen oder dermalen Verabreichung formuliert sein. Eine Verabreichung ist auch über ein Implantat oder ein semiautomatisches oder manuelles Schmerz-Pumpen-System möglich. Während eine lokale Verabreichung möglich ist, kommen die Vorteile der Erfindung, nämlich die Unterdrückung der zentralen Wirkung des Wirkstoffs, bei systemischer Verabreichung mehr zum Tragen.

Im Falle eines Kits kann dieses als selbstmischende oder vorgemischte zwei-Komponenten-Spritze vorliegen.

Die erfindungsgemäße pharmazeutische Zusammensetzung oder das pharmazeutische Kit ist zur Verwendung bei der Behandlung von akuten und chronischen Schmerzen und/oder Entzündungen geeignet. Besonders gut sind die erfindungsgemäßen Zusammensetzungen für die Behandlung von Schmerzen geeignet, die mit Entzündungen assoziiert sind.

Dabei werden durch eine Aktivierung peripherer Opioidrezeptoren Schmerzen reduziert, wobei aber ein Durchdringen der BHS im Vergleich zu einer entsprechenden Zusammensetzung (gleiche Konzentration des gleichen Wirkstoffs, ohne Nanocarrier, sonst gleiche Hilfsstoffe, gleiche Verabreichungsform) reduziert ist.

Die Kombination aus Nanocarrier und dem mit dem Opiodrezeptor wechselwirkenden Analgetikum, kovalenter oder nicht kovalenter Natur, wird im Rahmen der Erfindung auch als NANO-O bezeichnet. Sie weist ein hohes Molekulargewicht und Hydrophilie auf und wurde so maßgeschneidert, dass es den Wirkstoff im Wesentlichen im sauren, entzündeten und/oder verletzten Gewebe freisetzt, aber gleichzeitig die Permeation durch die BHS verhindert oder reduziert. Im Gegensatz zu herkömmlichen Opioiden, aktiviert intravenöses NANO-O im Wesentlichen periphere Opioidrezeptoren und erzeugt somit Analgesie in schmerzhaft entzündetem Gewebe, ohne dass zentrale Nebenwirkungen wie Sedierung und Atemdepression oder intestinale Nebenwirkungen (Konstipation) auftreten. Dabei sind gemessene Konzentrationen des Wirkstoffes im Gehirn und im Blut sehr gering, jedoch wird er im entzündeten Gewebe und bei saurem pH selektiv freigesetzt. NANO-O ist damit ein neuartige makromolekulare analgetische Wirkstoffklasse, die auf periphere Opioidrezeptoren in verletztem Gewebe wirkt, wobei zentrale oder intestinale Nebenwirkungen vermindert sind.

Erfindungsgemäß werden vorteilhafterweise durch Aktivierung von Opioidrezeptoren im intestinalen oder zentralen Nervensystem ausgelöste (zentrale) unerwünschte Wirkungen wie Sucht, Atemdepression, Übelkeit, Sedierung, Konstipation, neonatale Depression, Halluzinationen, und Depressions-ähnliche Symptome im Vergleich zu einer Zusammensetzung ohne Nanocarrier reduziert oder bevorzugt gar nicht auftreten.

Diese Patentanmeldung beschreibt also eine neue Verwendung einer Arzneimittelklasse, die nicht auf die zielgerichtete Wirkstofffreigabe über Gewebe-spezifische Antigene oder Rezeptoren abzielt. Die Erfinder machen sich dagegen die überraschende Erkenntnis zunutze, dass der Transfer von Analgetika, insbesondere Opioiden, durch die BHS bzw. und bevorzugt durch die Blutgefäßbarriere im nicht-entzündeten (unverletzten) Magen-Darm Trakt sowie durch die Plazentaschranke durch die erfindungsgemäß genutzten Nanocarrier blockiert wird. Ferner nutzen sie die Charakteristika der am weitesten verbreiteten Form klinischer Schmerzen, d.h. Schmerzen, die durch lokalisierte oder disseminierte Entzündungen hervorgerufen werden. Entzündungen gehen einher mit Gewebeübersäuerung (Azidose), die durch die Freisetzung von Protonen durch beschädigte Zellen verursacht wird. Gewebeazidose ist damit ein wesentlicher Bestandteil von vielen schmerzhaften Syndromen, wie z. B. Arthritis, Arthrose, Endometriose, Reizdarmsyndrom, neurogene Migräne, neuropathischen Schmerzen, Krebs, Trauma, Operationen und Wunden [Steen, K.H. et al., Pain 66, 163-170 (1996), Woo, Y.C. et al., Anesthesiology 101, 468-475 (2004); Stein, C. et al., Brain Res Rev 60, 90-113 (2009)]. Chronische-entzündliche Krankheiten gehören aktuell weltweit zu den häufigsten Gesundheitsbedrohungen [Tabas, I. et al., Science 339, 166-172 (2013)]. Weiterhin zeichnen sich entzündete Gewebe durch Fenestration sowie stärker durchlässige Kapillargefäße aus.

Das von uns gefundene NANO-O setzt selektiv peripher, und wegen der Charakteristika des Nanocarriers zudem bevorzugt in entzündetem Gewebe den Wirkstoff frei und erzeugt Analgesie durch die Aktivierung peripherer Opioidrezeptoren. Diese Wirkung kann sowohl über mu-, kappa- und delta-Opioidrezeptoren oder einer Mischung hieraus erfolgen.

Auf Grund der Unüberwindbarkeit der BHS ist es zu erwarten, dass bei mu-Rezeptor Agonisten bei bestimmungsgemäßer Anwendung keine Suchtgefahr besteht, sowie eine Gefahr der Gewöhnung auf Grund des peripher wirkenden Wirkmechanismus reduziert ist.

Eine Verknüpfung von Nanocarriern mit kappa-Opioidrezeptor-Agonisten erlaubt eine erleichterte Verschreibung und bessere Zugänglichkeit für Patienten in humanmedizinischen und veterinärmedizinischen Anwendungen, da eine Suchtgefahr auch bei nichtbestimmungsgemäßem Gebrauch signifikant vermindert ist. Insbesondere ist ein Missbrauch nach Extraktion des Wirkstoffes aus dem Medikament durch den Patienten ausgeschlossen, da in diesem Falle die dysphorische Nebenwirkung und aversive Nebenwirkung einer Suchtgefahr erfolgreich vorbeugt.

Um den erfindungsgemäßen Effekt hervorzurufen, sollte der Nanocarrier daher ausreichend in Blut- und Gewebeflüssigkeiten löslich sein, einen ausreichenden Übertritt des Wirkstoffes in das entzündete Gewebe ermöglichen und gleichzeitig den Übertritt des Wirkstoffes durch die BHS oder intestinale Blutgefäßbarriere ausreichend reduzieren, so dass die Wirkung des Wirkstoffes primär auf das periphere verletzte Gewebe beschränkt ist.

Die Erfindung stellt einen neuen Ansatz für die Entwicklung von optimierten Schmerzmitteln zur Verfügung. Die derzeit verfügbaren Schmerzmittel haben häufig schädliche Nebenwirkungen zur Folge [Schumacher, M.A. et al., in Basic and clinical pharmacology (eds. Katzung, B.G., Masters, S.B. & Trevor, A.J.) 531-552 (McGraw-Hill Medical, New York, 2009); Zollner, C. et al., Handb Exp Pharmacol, 31-63 (2007); Lancet 382:769-79, 2013]. Dagegen bietet die erfindungsgemäße Verwendung von Opioiden, die mit hydrophilen und bevorzugt positiv oder negativ geladenen Nanocarrier kombiniert, z.B. assoziiert, sind, eine neue Generation von Opioiden mit reduzierten zentralen oder intestinalen Nebenwirkungen. Insbesondere negativ geladenen Nanocarriern kommt hier eine besonders wichtige Rolle zu, da viele Opioide positive Ladungen tragen. Durch die erfindungsgemäße Darreichungsform kann ein Übertritt der Opioide über die BHS, Plazentaschranke oder intestinale Blutgefäßbarriere reduziert werden.

Solche selektiv peripher agierenden Opioid-Konjugate können im Vergleich zu klassischen Opioiden eine ähnliche schmerzlindernde Wirkung erreichen, ohne jedoch Barrieren wie Blut-Hirn-, Darm- oder Plazenta-Schranken zu durchdringen. Damit wären Nebenwirkungen wie Sedierung, Abhängigkeit, Verstopfung, Atemwegs- oder fötale/neonatale Depression ausgeschlossen [Steen, K.H. et al., Pain 66, 163-170 (1996), Woo, Y.C. et al., Anesthesiology 101, 468-475 (2004), Stein, C. et al., Brain Res Rev 60, 90-113 (2009), Tulamo, R.M., et al. Equine Vet J 21, 325-331 (1989), Guthrie, A.J. et al., J Vet Pharmacol Ther 19, 44-49 (1996), Bunczak-Reeh, M.A. et al., J Endod 24, 822-825 (1998). Auch eine analgetische Behandlung von schwangeren Frauen oder trächtigen Tieren ist mit der erfindungsgemäßen Zusammensetzung daher möglich.

Die Erfindung wird in den folgenden Beispielen weiter illustriert, soll jedoch nicht auf die Beispiele beschränkt sein. Die zitierten Referenzen werden durch die Bezugnahme vollumfänglich in die Anmeldung einbezogen.
**Fig. 1** **A** Struktur der drei ausgewählten Ankermoleküle. **B** Struktur von U50488. **C** Darstellung des mit N-Cbz-L-Asparaginsäure funktionalisierten Nanocarriers und der Wechselwirkungen mit dem Wirkstoff U50488 (rot: Salzbrücken, blau: π-π-Wechselwirkungen).
**Fig. 2** Analgetische Wirkungen von U50488 **(a)** und U50488-CMS **(b)** nach intraplantarer Injektion in die entzündete Pfote in Ratten mit unilateraler Hinterpfotenentzündung (Mittelwerte ± SEM; N = 3 Ratten pro Gruppe). Die Dosen wurden als freie Base berechnet.
**Fig. 3** Analgetische Wirkungen von U50488 **(a, b)** und U50488-CMS **(c, d)** nach intravenöser Injektion in Ratten mit unilateralen Hinterpfotenentzündung (Mittelwerte ± SEM; N = 2-3 Ratten pro Gruppe). Die Dosen wurden als freie Base berechnet.

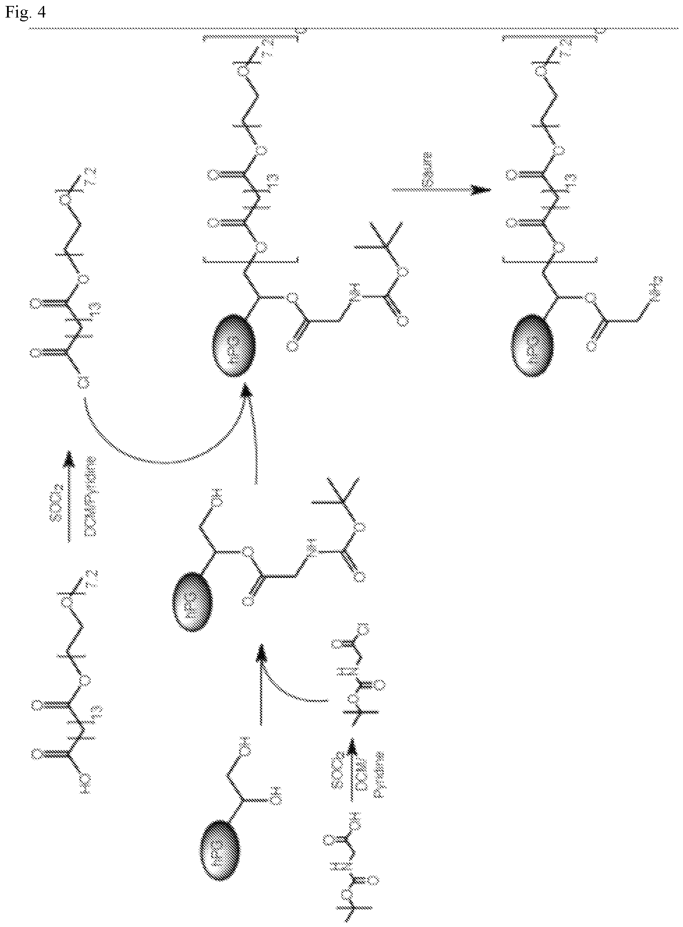

**Fig. 4** Beispielhafte Synthese eines erfindungsgemäß verwendeten Nanocarriers

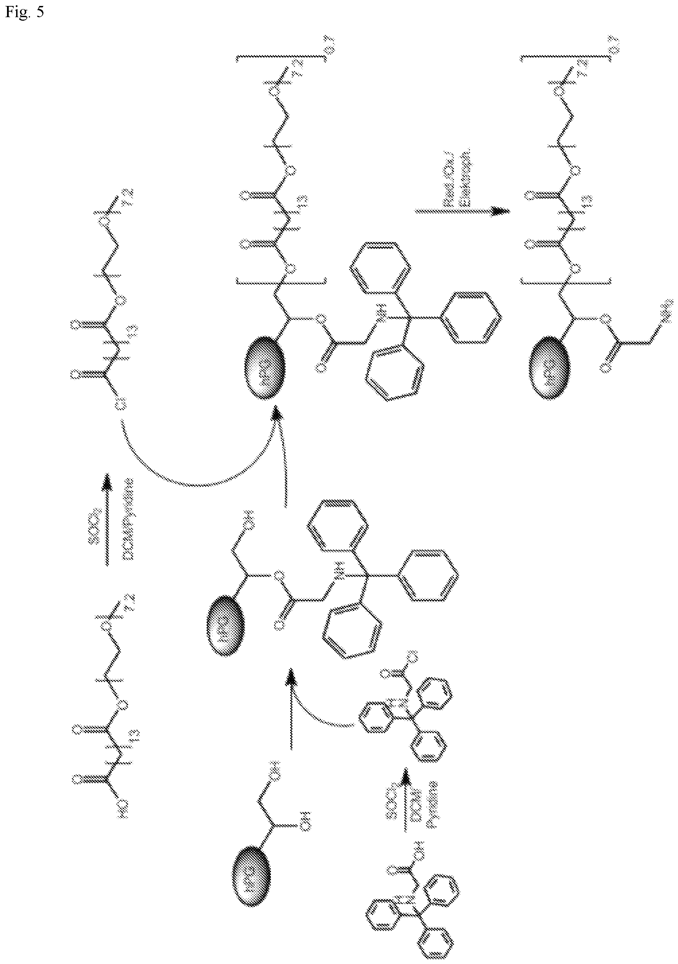

**Fig. 5** Beispielhafte Synthese eines erfindungsgemäß verwendeten Nanocarriers

### Beispiele

### 1. Herstellung von Nanocarriern

Als Nanocarrier für das unten beschriebene Beispiel wurde CMS-Amid-C18-mPEG350 verwendet. Dieser kann wie im Patent EP 1796649 synthetisiert werden. Dieser Carrier enthält freie positiv geladene Aminogruppen und wurde im Beispiel mit U50488-Sulfonat eingesetzt.

Als weitere Nanocarrier wurden solche vom Typ CMS Ester C15 mPEG350 verwendet. Dieser Carrier ist auf Grund seiner amphiphilen Eigenschaften gut geeignet, um sowohl hydrophile als auch hydrophobe Opioide zu verkapseln. Er ist gut wasserlöslich und polar. Er wird aus einem hyperverzweigten Polyglycerin-Kern (hPG) und einem mPEG-Dicarbonsäuremonoester synthetisiert. Beide Synthesen sind literaturbekannt [Sunder, A.H. et al., Macromolecules 32, 4240-4246 (1999); Sunder, A. et al., Chemistry 6, 2499-2506 (2000); Haag, R. et al., J Comb Chem 4, 112-119 (2002); Radowski, M. R. et al., Angew Chem Int Ed Engl 46, 1265-1269 (2007)]. Um den mPEG-Dicarbonsäuremonoester mit dem hPG-Kern zu verbinden wird dieser zunächst mit Thionylchlorid in Dichlormethan zum entsprechenden Säurechlorid umgesetzt. Anschließend wird das Säurechlorid mit dem hPG in Pyridin zum fertigen Nanocarrier umgesetzt. Die Aufreinigung des Nanocarriers findet durch Membranfiltration mit Membranen, die eine spezifische Molekulargewichtsausschlussgrenze haben, statt. Dieser Nanocarrier kann zusätzlich mit geeigneten Molekülen, die eine Wechselwirkung mit dem verwendeten Wirkstoff eingehen, ausgestattet werden. Dazu werden die Hydroxy-Gruppen am hPG teilweise mit diesen Molekülen vorab abreagiert. Als konkretes Beispiel verwenden wir hier Bernsteinsäureanhydrid. Das Bernsteinsäureanhydrid wird mit katalytischen Mengen von Dimethylaminopyridin und dem hPG in Pyridin gelöst und über eine Ringöffnungs-Reaktion an das hPG gebunden. Die hPG-Bernsteinsäure wird dann mit dem mPEG-Dicarbonsäuremonoester wie oben beschrieben umgesetzt. Die anschließende Aufreinigung erfolgt ebenfalls über Membranfiltration.

Eine weitere Möglichkeit, die Anzahl der freien negativ geladenen Carboxylatgruppen zu erhöhen, lässt sich durch kontrollierte Zugabe von Säure, Base oder eines geeigneten Enzyms, welches einen Teil der Esterbindungen dieses Nanocarriertyps spaltet, realisieren und dabei zielgenau auf die gewünschte Ladungsdichte einstellen. Gleichzeitig entstehen durch diese Reaktion "Taschen" im Nanocarrier, die eine verstärkte nicht-kovalente Wirkstoffassoziation fördern. Durch eine anschließende Neutralisation oder Inaktivierung des Enzyms wird diese Reaktion beendet. Die Aufreinigung des Produktes erfolgt über Diafiltration.

Ein weiteres Beispiel für einen gut zur Verkapselung geeigneten Carrier sind Nanocarrier vom Typ CMS Ester C15 mPEG 350 mit kovalent verknüpften Peptiden sowie Aminosäuren oder deren Derivate. Durch die Verwendung geeigneter Aminosäuren oder deren Derivate mit einer ionischen und/oder aromatischen Gruppe wie z.B. Asparaginsäure, Glycin, N,N-Dimethylglycin, Glutaminsäure, Phenylalanin oder auch geschützte Aminosäuren wie z.B. N-Cbz-L-Asparaginsäure, N-Cbz-L-Glutaminsäure werden zusätzliche ionische und/oder pi-pi Wechselwirkungen mit dem Wirkstoff ermöglicht. Diese Nanocarrier werden in einem sequentiellen, skalierbaren Mehrstufenprozess durchgeführt. Die Aufreinigung des Produktes erfolgt z.B. über Diafiltration.

### 2. Herstellung einer erfindungsgemäß verwendeten Zusammensetzung von Nanocarriern und Opioiden (NANO-O) ohne kovalente Verbindung

CMS Nancarrier vom Typ hPG (Mn 10k) (Octadecandisäure-mPEG350 ester)-Amid (Fuktionalisierungsgrad zwischen 55 und 75 %) und U50488 - Sulfonat wurden in 0,9 % NaCl im Massenverhältnis 1:4 gelöst.

Die Proben wurden bei 1200 rpm über 22h gerührt. Im Anschluss wurde eine Sterilfiltration (200nm RC) vorgenommen. Das NANO-O wird in Beispiel 3 eingesetzt.

### 3. Analgetische Wirkung einer erfindungsgemäß verwendeten Zusammensetzung

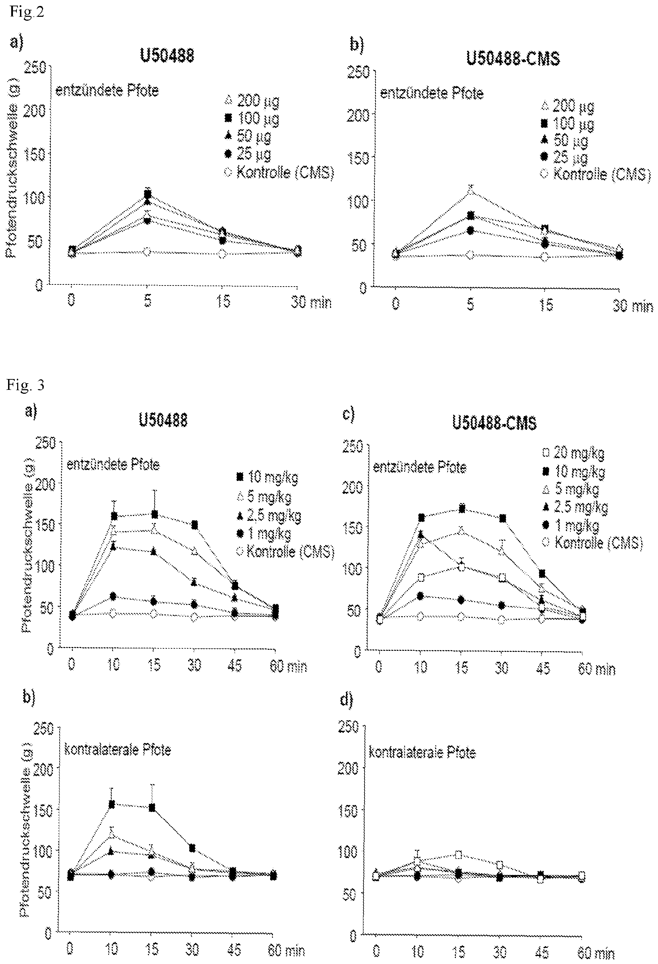

Zum Nachweis der selektiv in entzündetem Gewebe durch NANO-O hervorgerufenen Analgesie, wurde eine lokale Entzündung durch intraplantare (i.pl.) Injektion von 'komplettem Freund-Adjuvans' (CFA) in eine Hinterpfote von Ratten induziert [Stein, C. et al., Pharmacol Biochem Behav 31, 445-451 (1988)]. Nach vier Tagen wurde die sogenannte Pfotendruckschwelle (PPT) mittels eines Algesiometers gemessen. Die Pfotendruckschwelle bezeichnet den Druck, ab welchem die Ratte einen schmerzhaften Reiz empfindet und die Pfote zurückzieht. In unseren Experimenten wurde die Ausgangs-PPT in entzündeten Pfoten deutlich reduziert (Hyperalgesie), einhergehend mit früheren Studien [Stein, C. et al., Pharmacol Rev 63, 860-881 (2011); Stein, C. Anesth Analg 76, 182-191 (1993)] (niedrigere PPT in entzündeten Pfoten vs. PPT in nicht entzündeten, kontralateralen Pfoten. Es wurde die Auswirkung verschiedener Dosen (0-200 µg, berechnet als freie Base) von U50488 oder NANO-O in Form von U50488-CMS, jeweils i.pl. in die entzündeten Pfoten injiziert, untersucht. Dosis-abhängige PPT Erhöhung (Analgesie) wurde bereits 5-15 min nach erfolgter Injektion von bis zu 200 µg U50488 (Fig. 2a) oder U50488-CMS (Fig. 2b) in entzündeten Pfoten detektiert.

In den folgenden Versuchen wurde nun die Änderung der PPT nach intravenöser (i.v.) Injektion von NANO-O untersucht. Ab 10 min verursachte i.v. appliziertes U50488 einen Dosisabhängigen analgetischen Effekt sowohl in entzündeten als auch in nicht entzündeten Pfoten (Fig. 3a, b). Bei Applikation von 10 mg/kg U50488 war die Obergrenze für die PPT (ca. 160 g) in beiden Pfoten erreicht. Eine Verdopplung der Dosis auf 20 mg/kg war aus toxikologischen Gründen nicht möglich. Im Gegensatz dazu rief die i.v. Injektion von bis zu 10 mg/kg NANO-O eine analoge Dosis-abhängige analgetische Wirkung selektiv in entzündeten, jedoch nicht in kontralateralen, nicht-entzündeten Pfoten hervor (Fig. 3c, d). Bei Verabreichung von 20 mg/kg NANO-O war dieser analgetische Effekt auf Grund von Nebenwirkungen reduziert (Fig. 3c).

Nach Auswertung der analgetischen Wirksamkeit lässt sich zusammenfassend sagen, dass i.v. appliziertes NANO-O ausschließlich auf periphere Opioidrezeptoren in entzündetem Gewebe Wirkung zeigt, während i.v. U50488 ubiquitär sowohl periphere als auch zentrale Opioidrezeptoren aktiviert. Interessanterweise verursacht NANO-O keine zentrale analgetische oder sedierende Wirkung und signifikant verringerte systemische Toxizität.

### 4. Herstellung einer erfindungsgemäß verwendeten Zusammensetzung von Nanocarriern und Opioiden (NANO-O) mit kovalenter Verbindung

Ein Polymer-Wirkstoff-Konjugat mit analgetischer Wirkung kann über die kovalente Anbindung des Wirkstoffs, z.B. eines Opioids wie Nalbuphin, an das hochverzweigte Polyglycerin, unter Verwendung einer Glutarsäure-Gruppe, in einem zweistufigen Prozess hergestellt werden.

Das hochverzweigte Polyglycerin mit einem Molekulargewicht von 8-9 kDa, entsprechend einem Polymerisationsgrad (engl., degree of polymerization, DPₙ) von 108-121,5 Wiederholungseinheiten (engl., repeating unit, r.u.) wird über ein definiertes Monomer-Initiator-Verhältnis hergestellt. Über die Gleichung DPₙ = [M]/[I] ([M]: Stoffmenge Monomer, [I]: Stoffmenge Initiator) wird die benötigte Initiator bzw. Monomer-Menge berechnet. Die Beladung mit Glutarsäure-Gruppen wird über eine in situ Funktionalisierung des hochverzweigten Polyglycerins mit Glutarsäureanhydrid durchgeführt. Das Produkt wird z.B. über Diafiltration aufgereinigt. Das so erhaltene hochverzweigte Polyglycerin-Glutarat wird mit dem Wirkstoff, z.B. Nalbuphin, umgesetzt und erneut z.B. über Diafiltration aufgereinigt.

## Patentansprüche

1. Pharmazeutische Zusammensetzung, umfassend
- einen hydrophilen Nanocarrier, welcher ein hochverzweigtes Polymer ausgewählt aus der Gruppe umfassend dendritische Polymere und Dendrimere umfasst, wobei der dendritische Kern des Nanocarriers aus Polyglycerol ist und ein Molekulargewicht von 3-10 kDa hat, und
- ein Analgetikum, welches bei Verabreichung ohne Nanocarrier sowohl periphere Wirkung als auch Wirkung im Zentralnervensystem aufweist, welches ausgewählt ist aus der Gruppe bestehend aus Nalbuphin, Tramadol, Tilidin, Pethidin, Codein, Piritramid, Levomethadon, Fentanyl, Alfentanil, Remifentanil, Sufentanil, Pentazocin, Butorphanol, Buprenorphin, Methadon, 2-(3,4-dichlorophenyl)-N-methyl-N-[(1R,2R)-2-pyrrolidin-1-ylcyclohexyl]acetamid, (+)-(5α,7α,8β)-N-Methyl-N-[7-(1-pyrrolidinyl)-1-oxaspiro[4.5]dec-8-yl]-2-Phenylacetamid, Dimethyl 3,7-dimethyl-9-oxo-2,4-dipyridin-2-yl-3,7-diazabicyclo[3.3.1]nonane-1,5-dicarboxylat, 2-(3,4-dichlorophenyl)-1-[(2S)-2-(pyrrolidin-1-ylmethyl)piperidin-1-yl]ethanon, Bremazocine und Moxazozine,
zur Verwendung bei der Behandlung von akuten und chronischen Schmerzen und/oder Entzündungen durch periphere Wirkung des Analgetikums, wobei die Wirkung des Analgetikums im Zentralnervensystem im Vergleich zu einer entsprechenden Zusammensetzung ohne Nanocarrier reduziert ist.

2. Pharmazeutische Zusammensetzung nach Anspruch 1
zur Verwendung bei der Behandlung von Schmerzen, die mit Entzündungen assoziiert sind.

3. Pharmazeutische Zusammensetzung zur Verwendung nach einem der Ansprüche 1 oder 2, wobei durch eine Aktivierung peripherer Opioidrezeptoren Schmerzen reduziert werden, und wobei ein Durchdringen der Blut-Hirnschranke im Vergleich zu einer entsprechenden Zusammensetzung ohne Nanocarrier reduziert ist.

4. Pharmazeutische Zusammensetzung zur Verwendung nach einem der Ansprüche 1-3, wobei durch Aktivierung von Opioidrezeptoren im zentralen Nervensystem ausgelöste unerwünschte Wirkungen ausgewählt aus der Gruppe von Sucht, Atemdepression, Übelkeit, Sedierung, Konstipation, neonatale Depression, Halluzinationen, Depressions-ähnliche Symptome im Vergleich zu einer entsprechenden Zusammensetzung ohne Nanocarrier vermindert oder bevorzugt gar nicht auftreten.

5. Pharmazeutische Zusammensetzung zur Verwendung nach einem der Ansprüche 1-4, welche zur intravenösen, intramuskulären, intraperitonealen, subkutanen, pulmonaren oder dermalen Verabreichung formuliert ist.

6. Pharmazeutische Zusammensetzung zur Verwendung nach einem der Ansprüche 1-5, wobei der Nanocarrier ein hochverzweigtes dendritisches Polyglycerin-Polymer ist.

7. Ein Kit, umfassend
- einen hydrophilen Nanocarrier, welcher ein hochverzweigtes Polymer ausgewählt aus der Gruppe umfassend dendritische Polymere und Dendrimere umfasst, wobei der Nanocarrier ein hochverzweigtes dendritisches Polyglycerin-Polymer ausgewählt aus der Gruppe umfassend Kern-Schale-Nanocarrier und Kern-Multischalen-Nanocarrier ist, wobei der dendritische Kern des Nanocarriers aus Polyglycerol ist und ein Molekulargewicht von 3-10 kDa hat, und
- ein Analgetikum, welches bei Verabreichung ohne Nanocarrier sowohl periphere als auch zentrale analgetische Wirkung aufweist und ausgewählt ist aus der Gruppe bestehend aus Nalbuphin, Tramadol, Tilidin, Pethidin, Codein, Piritramid, Levomethadon, Fentanyl, Alfentanil, Remifentanil, Sufentanil, Pentazocin, Butorphanol, Buprenorphin, Methadon, 2-(3,4-dichlorophenyl)-N-methyl-N-[(1R,2R)-2-pyrrolidin-1-ylcyclohexyl]acetamid, (+)-(5α,7α,8β)-N-Methyl-N-[7-(1-pyrrolidinyl)-1-oxaspiro[4.5]dec-8-yl]-2-Phenylacetamid, Dimethyl 3,7-dimethyl-9-oxo-2,4-dipyridin-2-yl-3,7-diazabicyclo[3.3.1]nonane-1,5-dicarboxylat, 2-(3,4-dichlorophenyl)-1-[(2S)-2-(pyrrolidin-1-ylmethyl)piperidin-1-yl]ethanon, Bremazocine und Moxazozine,
zur Verwendung bei der Behandlung von akuten und chronischen Schmerzen und/oder Entzündungen durch periphere Wirkung des Analgetikums, wobei die Wirkung des Analgetikums im Zentralnervensystem im Vergleich zu einer entsprechenden Zusammensetzung ohne Nanocarrier reduziert ist.

8. Pharmazeutische Zusammensetzung oder Kit zur Verwendung nach einem der vorhergehenden Ansprüche, wobei das Analgetikum ein Opioid ist, welches eine agonistische oder partiell agonistische Wirkung an einem kappa-Opioid-Rezeptor aufweist.

9. Pharmazeutische Zusammensetzung oder Kit zur Verwendung nach einem der vorhergehenden Ansprüche, wobei Analgetikum und Nanocarrier kovalent miteinander assoziiert sind.

10. Pharmazeutische Zusammensetzung oder Kit zur Verwendung nach einem der vorhergehenden Ansprüche, wobei der Nanocarrier ein hochverzweigtes dendritisches Polyglycerin-Polymer ausgewählt aus der Gruppe bestehend aus Kern-Schale-Nanocarrier und Kern-Multischalen-Nanocarrier ist,
wobei der Nanocarrier bevorzugt aus einem dendritischen Kern und aus mindestens zwei Schalen besteht, wobei eine innere Schale mit einem ersten Linker mit dem dendritischen Kern verbunden ist und eine äußere Schale mit einem zweiten Linker mit der inneren Schale verbunden ist.

11. Pharmazeutische Zusammensetzung oder das Kit zur Verwendung nach einem der vorhergehenden Ansprüche, wobei der Nanocarrier ein Kern-Multischalen-Nanocarrier mit positiver oder negativer Ladung aus einem hochverzweigten dendritischen Polyglycerin-Polymer ist,
a) welcher durch ein Verfahren erhältlich ist, das Schritte umfasst, bei denen man die Ladung durch partielle Spaltung des Linkers zwischen innerer und äußerer Schale erzeugt, oder
b) welcher am Kern mit zusätzlichen Ankermolekülen, bevorzugt freien Säuregruppen ausgestattet ist, welche mit einer Verbindung ausgewählt aus der Gruppe umfassend Bernsteinsäureanhydrid, Glutarsäureanhydrid, Peptiden, Aminosäuren oder Aminosäurederivaten funktionalisiert sind, oder
c) welcher mit einer eine Aminogruppe umfassenden Verbindung ausgestattet ist.

12. Pharmazeutische Zusammensetzung oder das Kit zur Verwendung nach einem der vorhergehenden Ansprüche, wobei der Nanocarrier positive oder negative Ladungen aufweist, bevorzugt negative Ladungen.

13. Pharmazeutische Zusammensetzung oder das Kit zur Verwendung nach einem der vorhergehenden Ansprüche, wobei der Transfer des Analgetikums durch die Blut-Hirn-Schranke durch die Nanocarrier blockiert wird.

14. Pharmazeutische Zusammensetzung oder das Kit zur Verwendung nach einem der vorhergehenden Ansprüche, wobei die periphere Wirkung des Analgetikums nicht auf einer zielgerichteten Wirkstofffreigabe über Gewebe-spezifische Antigene oder Rezeptoren beruht.

## Claims

1. A pharmaceutical composition comprising
- a hydrophilic nanocarrier comprising a hyperbranched polymer selected from the group comprising dendritic polymers and dendrimers, wherein the dendritic core of the nanocarrier is from polyglycerol and has a molecular weight of 3-10 kDa, and
- an analgesic which when administered without nanocarrier has both peripheral action and action in the central nervous system, selected from the group consisting of nalbuphine, tramadol, tilidine, pethidine, codeine, piritramide, levomethadone, fentanyl, alfentanil, remifentanil, sufentanil, pentazocine, butorphanol, buprenorphine, methadone, 2-(3,4-dichlorophenyl)-N-methyl-N-[(1R,2R)-2-pyrrolidin-1-ylcyclo-hexyl]acetamide, (+)-(5α,7α,8β)-N-Methyl-N-[7-(1-pyrrolidinyl)-1-oxaspiro[4.5]dec-8-yl]-benzeneacetamide, dimethyl 3,7-dimethyl-9-oxo-2,4-dipyridin-2-yl-3,7-diazabicyclo[3.3.1]nonane-1,5-dicarboxylat, 2-(3,4-dichlorophenyl)-1-[(2S)-2-(pyrrolidin-1-ylmethyl)piperidin-1-yl]ethanone, bremazocine and moxazozine,
for use in the treatment of acute and chronic pain and/or inflammations through the peripheral action of the analgesic, wherein the effect of the analgesic is reduced in the central nervous system in comparison to a corresponding composition without nanocarrier.

2. The pharmaceutical composition for use of claim 1 for use in the treatment of pain associated with inflammation.

3. The pharmaceutical composition for use of any of claims 1 or 2, wherein pain is reduced through activation of peripheral opioid receptors, and wherein the penetration of the blood-brain barrier is reduced in comparison to a corresponding composition without nanocarrier.

4. The pharmaceutical composition for use of any of claims 1-3, wherein adverse effects elicited by activation of opioid receptors in the central nervous system selected from the group of addiction, respiratory depression, nausea, sedation, constipation, neonatal depression, hallucinations, depression-like symptoms, are reduced in comparison to a corresponding composition without nanocarrier, or, preferably, do not occur.

5. The pharmaceutical composition for use of any of claims 1-4, formulated for intravenous, intramuscular, intraperitoneal, subcutaneous, pulmonary or dermal administration.

6. The pharmaceutical composition for use of any of claims 1-5, wherein the nanocariier is a hyperbranched dendritic polyglycerol polymer.

7. A kit, comprising
- a hydrophilic nanocarrier comprising a hyperbranched polymer selected from the group comprising dendritic polymers and dendrimers, wherein the dendritic core of the nanocarrier is from polyglycerol and has a molecular weight of 3-10 kDa, and
- an analgesic which when administered without nanocarrier has both peripheral action and action in the central nervous system, selected from the group consisting of nalbuphine, tramadol, tilidine, pethidine, codeine, piritramide, levomethadone, fentanyl, alfentanil, remifentanil, sufentanil, pentazocine, butorphanol, buprenorphine, methadone, 2-(3,4-dichlorophenyl)-N-methyl-N-[(1R,2R)-2-pyrrolidin-1-ylcyclohexyl]acetamide, (+)-(5α,7α,8β)-N-Methyl-N-[7-(1-pyrrolidinyl)-1-oxaspiro[4.5]dec-8-yl]-benzeneacetamide, dimethyl 3,7-dimethyl-9-oxo-2,4-dipyridin-2-yl-3,7-diazabicyclo[3.3.1]nonane-1,5-dicarboxylat, 2-(3,4-dichlorophenyl)-1-[(2S)-2-(pyrrolidin-1-ylmethyl)piperidin-1-yl]ethanone, bremazocine and moxazozine,
for use in the treatment of acute and chronic pain and/or inflammations through the peripheral action of the analgesic, wherein the effect of the analgesic is reduced in the central nervous system in comparison to a corresponding composition without nanocarrier.

8. The pharmaceutical composition or the kit for use of any of the preceding claims, wherein the analgesic is an opioid having an agonist or partial agonist activity on kappa opioid receptor.

9. The pharmaceutical composition or the kit for use of any of the preceding claims, wherein the analgesic and nanocarrier are covalently associated with each other.

10. The pharmaceutical composition or the kit for use of any of the preceding claims, wherein the nanocarrier is a hyperbranched dendritic polyglycerol polymer selected from the group consisting of core-shell nanocarriers and core-multi-shell nanocarriers,
wherein the nanocarrier preferably consists of a dendritic core and of at least two shells, wherein the inner shell is connected to the dendritic core via a first linker and an outer shell is connected to the inner shell via a second linker.

11. The pharmaceutical composition or the kit for use of any of the preceding claims, wherein the nanocarrier is a core multi-shell nanocarrier having a positive or negative charge based on a hyperbranched dendritic polyglycerol polymer,
a) which is obtainable by a method comprising steps of generating the charge by a partial cleavage of the linker between the inner and outer shell, or
b) which is equipped at the core with additional anchor molecules, preferably free acid groups, which are functionalized with a compound selected from the group consisting of succinic anhydride, glutaric anhydride, peptides, amino acids or amino acid derivatives, or
c) which is equipped with a compound containing an amino group.

12. The pharmaceutical composition or the kit for use of any of the preceding claims, wherein the nanocarrier has positive or negative charges, preferably, negative charges.

13. The pharmaceutical composition or the kit for use of any of the preceding claims, wherein the transfer of the analgesic through the blood-brain barrier is blocked by the nanocarrier.

14. The pharmaceutical composition or the kit for use of any of the preceding claims, wherein the peripheral effect of the analgesic is not based on a targeted release of active agent through tissue-specific antigens or receptors.

## Revendications

1. Composition pharmaceutique comprenant :
- un nano-support hydrophile qui comprend un polymère hautement ramifié, choisi dans l'ensemble comprenant les polymères dendriti-ques et les dendrimères, duquel nano-support le noyau dendritique est à base de polyglycérol et présente une masse molaire valant de 3 à 10 kDa,
- et un analgésique qui, lorsqu'on l'administre sans nano-support, exerce aussi bien une action périphérique qu'une action au niveau du système nerveux central, et qui est choisi dans l'ensemble formé par les nalbuphine, tramadol, tilidine, péthidine, codéine, piritramide, lévométhadone, fentanyl, alfentanil, rémifentanil, sufentanil, pentazocine, butorphanol, buprénorphine, méthadone, 2-(3,4-dichloro-phényl)-N-méthyl-N-[(1R,2R)-2-pyrrolidin-1-yl-cyclohexyl]-acétamide, (+)-(5α,7α,8β)-N-méthyl-N-[7-(1-pyrrolidinyl)-1-oxaspiro[4.5]dec-8-yl]-benzèneacétamide, 3,7-diméthyl-9-oxo-2,4-dipyridin-2-yl-3,7-diaza-bicyclo[3.3.1]nonane-1,5-dicar-boxylate de diméthyle, 2-(3,4-dichloro-phényl)-1-[(2S)-2-(pyrro-lidin-1-yl-méthyl)-pipéridin-1-yl]-éthanone, brémazocine et moxazocine,
pour utilisation dans le traitement de douleurs et/ou inflammations aiguës et chroniques par l'action périphérique de l'analgésique, l'action de l'analgésique sur le système nerveux central étant réduite, comparée à celle d'une composition correspondante sans nano-support.

2. Composition pharmaceutique conforme à la revendication 1, pour utilisation dans le traitement de douleurs qui sont associées à des inflammations.

3. Composition pharmaceutique pour utilisation conforme à l'une des revendications 1 et 2, avec laquelle les douleurs sont réduites par activation de récepteurs opioïdes périphériques, et la traversée de la barrière hémato-encéphalique est réduite, comparée au cas d'une composition correspondante sans nano-support.

4. Composition pharmaceutique pour utilisation conforme à l'une des revendications 1 à 3, avec laquelle des effets indésirables sus-cités par l'activation des récepteurs opioïdes dans le système nerveux central, choisis dans l'ensemble comprenant dépendance, dépression respiratoire, nausée, sédation, constipation, dépression post-partum, hallucinations et symptômes dépressionnaires, sont diminués, comparés à ceux d'une composition correspondante sans nano-support, ou de pré-férence n'apparaissent pas du tout.

5. Composition pharmaceutique pour utilisation conforme à l'une des revendications 1 à 4, qui est formulée pour administration par voie intraveineuse, intramusculaire, intrapéritonéale, sous-cutanée, pulmonaire ou dermique.

6. Composition pharmaceutique pour utilisation conforme à l'une des revendications 1 à 5, dans laquelle le nano-support est un polymère polyglycérine dendritique hautement ramifié.

7. Kit comprenant :
- un nano-support hydrophile qui comprend un polymère hautement ramifié, choisi dans l'ensemble comprenant les polymères dendriti-ques et les dendrimères, lequel nano-support est un polymère poly-glycérine dendritique hautement ramifié, choisi dans l'ensemble comprenant un nano-support noyau-coque et un nano-support noyau-multicoque, et duquel nano-support le noyau dendritique est à base de polyglycérol et présente une masse molaire valant de 3 à 10 kDa,
- et un analgésique qui, lorsqu'on l'administre sans nano-support, exerce une action analgésique tant périphérique que centrale et qui est choisi dans l'ensemble formé par les nalbuphine, tramadol, tilidine, péthidine, codéine, piritramide, lévométhadone, fentanyl, alfentanil, rémifentanil, sufentanil, pentazocine, butorphanol, buprénorphine, méthadone, 2-(3,4-dichloro-phényl)-N-méthyl-N-[(1R,2R)-2-pyrrolidin-1-yl-cyclohexyl]-acétamide, (+)-(5α,7α,8β)-N-méthyl-N-[7-(1-pyrrolidinyl)-1-oxaspiro[4.5]dec-8-yl]-benzèneacétamide, 3,7-diméthyl-9-oxo-2,4-dipyridin-2-yl-3,7-diaza-bicyclo[3.3.1]nonane-1,5-dicar-boxylate de diméthyle, 2-(3,4-dichloro-phényl)-1-[(2S)-2-(pyrro-lidin-1-yl-méthyl)-pipéridin-1-yl]-éthanone, brémazocine et moxazocine,
pour utilisation dans le traitement de douleurs et/ou inflammations aiguës et chroniques par l'action périphérique de l'analgésique, l'action de l'analgésique sur le système nerveux central étant réduite, comparée à celle d'une composition correspondante sans nano-support.

8. Composition pharmaceutique ou kit pour utilisation, con-forme à l'une des revendications précédentes, dans laquelle ou lequel l'analgésique est un opioïde qui exerce un effet agoniste ou partielle-ment agoniste sur un récepteur opioïde kappa.

9. Composition pharmaceutique ou kit pour utilisation, con-forme à l'une des revendications précédentes, dans laquelle ou lequel l'analgésique et le nano-support sont associés l'un à l'autre en mode covalent.

10. Composition pharmaceutique ou kit pour utilisation, con-forme à l'une des revendications précédentes, dans laquelle ou lequel le nano-support est un polymère polyglycérine dendritique hautement ramifié, choisi dans l'ensemble comprenant un nano-support noyau-coque et un nano-support noyau-multicoque,
lequel nano-support est de préférence constitué d'un noyau dendritique et d'au moins deux coques, avec une coque interne liée au noyau dendritique au moyen d'un premier raccord et une coque externe liée à la coque interne au moyen d'un deuxième raccord.

11. Composition pharmaceutique ou kit pour utilisation, con-forme à l'une des revendications précédentes, dans laquelle ou lequel le nano-support est un nano-support noyau-multicoque porteur d'une charge positive ou négative et constitué d'un polymère polyglycérine dendritique hautement ramifié,
a) qui est accessible par un procédé comportant une étape dans laquelle la charge est produite par scission partielle du raccord entre les couches interne et externe,
b) ou qui est muni, au niveau du noyau, de molécules d'ancrage supplémentaires, de préférence de groupes acides libres, qui sont fonctionnalisées avec un composé choisi dans l'ensemble compre-nant l'anhydride succinique, l'anhydride glutarique et les peptides et acides aminés ou dérivés d'acide aminé,
c) ou qui est muni d'un composé comportant un groupe amino.

12. Composition pharmaceutique ou kit pour utilisation, con-forme à l'une des revendications précédentes, dans laquelle ou lequel le nano-support porte des charges positives ou négatives, et de préfé-rence, des charges négatives.

13. Composition pharmaceutique ou kit pour utilisation, con-forme à l'une des revendications précédentes, avec laquelle ou lequel le transport de l'analgésique à travers la barrière hémato-encéphalique est empêché par le nano-support.

14. Composition pharmaceutique ou kit pour utilisation, con-forme à l'une des revendications précédentes, avec laquelle ou lequel l'action périphérique de l'analgésique ne repose pas sur une libération ciblée de substance active par l'intermédiaire d'antigènes ou de récep-teurs spécifiques de tissu.
